# EUROPEAN PATENT SPECIFICATION

(11) **EP 4 683 526 B1**
(45) Date of publication and mention of the grant of the patent: **08.07.2026**
(21) Application number: 24718702.4
(22) Date of filing: 03.04.2024
(51) Int. Cl.: A24B 13/00, A24B 15/42

(54) **POUCH WITH CROSSLINKED ALGINATE**
BEUTEL MIT VERNETZTEM ALGINAT
SACHET À ALGINATE RÉTICULÉ

(30) Priority: 03.04.2023 DK PA202370168
(43) Date of publication of application: 28.01.2026
(73) Proprietor: Fertin Pharma A/S, 7100 Vejle (DK)
(72) Inventor: STAHL, My Ly Lao, 7100 Vejle (DK)
(74) Representative: Patentgruppen A/S
(86) International application number: PCT/DK2024/050079
(87) International publication number: WO 2024/208402

(56) References cited:
- EP-A1- 4 144 231
- EP-B1- 4 000 424
- WO-A1-2022/100805
- US-A1- 2022 152 013
- US-B2- 11 406 630

## Description

### FIELD OF INVENTION

The invention relates to an oral nicotine pouch composition according to the claims and a nicotine pouched product according to the claims.

### BACKGROUND

In the latest years a wide range of nicotine products having a reduced tobacco content or without tobacco has been seen, also within the field of pouched products or pouches. Oral nicotine pouched compositions are disclosed in documents WO2022/100805 A1 and EP4000424 B1.

Yet for pouched products, it remains a desire to provide a faster nicotine release in order to give the user an improved experience by means of faster craving relief, while at the same time providing a desirable user experience.

### SUMMARY

The invention relates to an oral nicotine pouch composition comprising
nicotine,
water in an amount of at least 5% by weight of the pouch composition,
alginate,
inorganic divalent cations, and
at least one pH regulating agent comprising inorganic phosphate.

An advantage of the invention may be that the use of pH regulating agent comprising inorganic phosphate may provide an increase of the pH value of the user, which facilitates effective nicotine uptake. At the same time, an improvement in taste may be obtained, since off notes associated with other pH regulating agents may be reduced. Combining with the use of alginate that facilitates preserving the water content above a predetermined level in order to give a desirable mouthfeel, the pouch composition may be desirable for the user due to an improved mouthfeel and also provide an effective craving relief.

Surprisingly, the present inventor found that while obtaining the above advantages, a considerable improvement in processability may be obtained. Typically, the use of water in the pouch composition may result in undesirable properties with respect to processability as lumping may occur and the flow properties may get worse. The present invention surprisingly provides an improved processability while retaining advantages associated with inclusion of water in the pouch composition and the use of alginate.

In the present context it is noted that the term "inorganic divalent cations" refers to a single type of divalent cations or to a mixture of two or more types of divalent cations. For example, two different types of divalent cations may be provided as salts with the same or different counterion, for example as a combination of calcium chloride and magnesium chloride, or as a combination of calcium chloride and magnesium bromide. Also, the same type of inorganic divalent cations may be provided as two or more different salts, i.e. with two or more different counterions, for example calcium chloride and calcium bromide.

In the present context it is noted that the term "phosphate" refers to monophosphate, polyphosphate, such as pyrophosphate (also known as diphosphate), triphosphate, tetraphosphate etc., and any combinations thereof. It is noted that when referring to certain salts, such as e.g. sodium salts, such reference refers to any sodium salt of the particular phosphate, such that e.g. sodium pyrophosphate refers also to tetrasodium pyrophosphate.

In the present context the term "alginate" refers to the ionic form of alginate, e.g. in the form of a salt, as well as alginic acid.

According to an advantageous embodiment of the invention, the phosphate comprises at least two different phosphates.

According to an embodiment of the invention, the phosphate consists of a single phosphate.

According to an advantageous embodiment of the invention, the phosphate comprises monophosphate.

According to an advantageous embodiment of the invention, the phosphate comprises polyphosphate, such as pyrophosphate.

According to an advantageous embodiment of the invention, the phosphate comprises a combination of monophosphate and polyphosphate, such as pyrophosphate.

In an embodiment of the invention, the phosphate is a combination of monophosphate and polyphosphate, such as pyrophosphate.

According to an advantageous embodiment of the invention, the phosphate comprises phosphate provided as a salt with an alkali metal, such as sodium or potassium.

In an embodiment of the invention, the phosphate is provided as a salt with an alkali metal.

According to an embodiment of the invention, the phosphate comprises phosphate provided as a salt with sodium.

According to an advantageous embodiment of the invention, the phosphate is provided as a salt with sodium.

According to an advantageous embodiment of the invention, the phosphate comprises monosodium phosphate.

According to an advantageous embodiment of the invention, the phosphate comprises disodium phosphate.

According to an advantageous embodiment of the invention, the phosphate comprises trisodium phosphate.

According to an advantageous embodiment of the invention, the phosphate comprises monosodium diphosphate.

According to an advantageous embodiment of the invention, the phosphate comprises disodium diphosphate.

According to an advantageous embodiment of the invention, the phosphate comprises trisodium diphosphate.

According to an advantageous embodiment of the invention, the phosphate comprises tetrasodium diphosphate.

In preferred embodiments of the invention the phosphate is anhydrous.

In embodiments of the invention the phosphate comprises hydrated phosphate, i.e. the phosphate comprises crystal bound water.

According to an embodiment of the invention, the phosphate comprises phosphate provided as a salt with potassium.

According to an advantageous embodiment of the invention, the phosphate is provided as a salt with potassium.

In an embodiment of the invention, the phosphate is provided as a water-soluble salt having a water-solubility of at least 5 gram per 100 mL of water measured at 25 degrees Celsius, atmospheric pressure and pH 7.0, such as 5 to 500 gram per 100 mL of water.

According to an advantageous embodiment of the invention, the phosphate is provided as a salt in an amount of at least 0.1% by weight of the pouch composition, such as at least 0.2% by weight of the pouch composition, such as at least 0.5% by weight of the pouch composition, such as at least 1.0% by weight of the pouch composition.

In an embodiment of the invention, the phosphate is provided as a salt in an amount of no more than 7.0% by weight of the pouch composition, such as no more than 5.0% by weight of the pouch composition, such as no more than 4.5% by weight of the pouch composition, such as no more than 3.0% by weight of the pouch composition, such as no more than 2.0% by weight of the pouch composition.

In an embodiment of the invention, the phosphate is provided as a salt in an amount of 0.1 to 7.0% by weight of the pouch composition, such as 0.1 to 5.0% by weight of the pouch composition, such as 0.2 to 4.5% by weight of the pouch composition, such as 0.5 to 3.0% by weight of the pouch composition, such as 1.0 to 2.0% by weight of the pouch composition.

According to an advantageous embodiment of the invention, the pouch composition comprises alginate in an amount of at least 0.1% by weight of the pouch composition, such as at least 0.2% by weight of the pouch composition, such as at least 0.3% by weight of the pouch composition, such as at least 0.4% by weight of the pouch composition.

In the present context it should be understood that the amount of alginate refers to the amounts (weights) of alginate compounds i.e. the alginate ion with its counterion.

In an embodiment of the invention, the pouch composition comprises alginate in an amount of no more than 5.0% by weight of the pouch composition, such as no more than 3.0% by weight of the pouch composition, such as no more than 2.0% by weight of the pouch composition, such as no more than 1.5% by weight of the pouch composition, such as no more than 1.0% by weight of the pouch composition.

In an embodiment of the invention, the pouch composition comprises alginate in an amount of 0.1 to 5.0% by weight of the pouch composition, such as 0.1 to 3.0% by weight of the pouch composition, such as 0.2 to 2.0% by weight of the pouch composition, such as 0.3 to 1.5% by weight of the pouch composition, such as 0.4 to 1.0% by weight of the pouch composition.

According to an advantageous embodiment of the invention, the alginate is provided as a salt with an ion selected from the group consisting of alkali metal alginate, such as sodium alginate or potassium alginate, or any combinations thereof.

In an embodiment of the invention, the alginate comprises alginate selected from the group consisting of sodium alginate, potassium alginate, and any combination thereof.

In an embodiment of the invention, the alginate is selected from the group consisting of sodium alginate, potassium alginate, and any combination thereof.

In an embodiment of the invention, the alginate comprises sodium alginate.

In an embodiment of the invention, the alginate is sodium alginate.

In an embodiment of the invention, the alginate comprises potassium alginate.

In an embodiment of the invention, the alginate is potassium alginate.

According to an advantageous embodiment of the invention, the inorganic divalent cations are selected from the group consisting of divalent cations of calcium, magnesium, iron, zinc, and any combination thereof.

According to an advantageous embodiment of the invention, the inorganic divalent cations are selected from the group consisting of divalent cations of calcium, magnesium, and any combination thereof.

According to an advantageous embodiment of the invention, the inorganic divalent cations comprise calcium ions.

In an embodiment of the invention, the inorganic divalent cations consist of calcium ions.

According to an advantageous embodiment of the invention, the inorganic divalent cations comprise magnesium ions.

In an embodiment of the invention, the inorganic divalent cations consist of magnesium ions.

According to an embodiment of the invention, the divalent cations are provided as a salt.

According to an advantageous embodiment of the invention, the divalent cations are provided as a water-soluble salt having a water-solubility of at least 5 gram per 100 mL of water measured at 25 degrees Celsius, atmospheric pressure and pH 7.0.

Thus, in the present context the term water-soluble salt refers to salt having a water-solubility of at least 5 gram per 100 mL of water measured at 25 degrees Celsius, atmospheric pressure and pH 7.0.

In an embodiment of the invention, the divalent cations are provided as a water-soluble salt having a water-solubility of 5 to 500 gram per 100 mL of water measured at 25 degrees Celsius, atmospheric pressure and pH 7.0, such as 5 to 350 gram per 100 mL of water measured at 25 degrees Celsius, atmospheric pressure and pH 7.0.

According to an advantageous embodiment of the invention, the inorganic divalent cations are provided as a salt comprising anions selected from the group consisting of carboxylates, such as acetate, lactate, oxalate, propionate, or levulinate; organic sulfonate; organic sulfate; organic phosphate; chloride, bromide, nitrate, sulfate, hydrogen phosphate, oxide, and any combination thereof.

According to an advantageous embodiment of the invention, the inorganic divalent cations are provided as an inorganic salt.

According to an advantageous embodiment of the invention, the inorganic divalent cations are provided as an inorganic salt comprising inorganic anions selected from the group consisting of chloride, bromide, nitrate, sulfate, hydrogen carbonate, hydrogen phosphate, oxide, hydroxide, and any combination thereof.

According to an advantageous embodiment of the invention, the inorganic divalent cations are provided as an inorganic salt comprising inorganic anions selected from the group consisting of chloride, bromide, hydrogen carbonate, sulfate, and any combination thereof.

According to an advantageous embodiment of the invention, the inorganic divalent cations are provided as an inorganic salt comprising inorganic anions are selected from the group consisting of chloride, bromide, sulfate and any combination thereof.

According to an advantageous embodiment of the invention, the divalent cation is provided as an inorganic salt with chloride.

According to an advantageous embodiment of the invention, the divalent cation is provided as an inorganic salt with bromide.

According to an advantageous embodiment of the invention, the divalent cation is provided as an inorganic salt with sulfate.

According to an advantageous embodiment of the invention, the divalent cations are selected from the group consisting of calcium chloride, calcium bromide, calcium sulfate, magnesium chloride, magnesium bromide, magnesium sulfate, and any combination thereof.

According to an embodiment of the invention, the divalent cations comprise divalent cations provided as calcium chloride.

According to an embodiment of the invention, the divalent cations are provided as calcium chloride.

According to an embodiment of the invention, the divalent cations comprise divalent cations provided as calcium bromide.

According to an embodiment of the invention, the divalent cations are provided as calcium bromide.

According to an embodiment of the invention, the divalent cations comprise divalent cations provided as calcium sulfate.

According to an embodiment of the invention, the divalent cations are provided as calcium sulfate.

According to an advantageous embodiment of the invention, the inorganic divalent cations are provided as a salt in an amount of at least 0.1% by of the pouch composition, such as at least 0.2% by weight of the pouch composition, such as at least 0.3% by weight of the pouch composition, such as at least 0.4% by weight of the pouch composition.

In an embodiment of the invention, the inorganic divalent cations are provided as a salt in an amount of no more than 5.0% by weight of the pouch composition, such as no more than 3.0% by weight of the pouch composition, such as no more than 2.0% by weight of the pouch composition, such as no more than 1.5% by weight of the pouch composition, such as no more than 1.0% by weight of the pouch composition.

In an embodiment of the invention, the inorganic divalent cations are provided as a salt in an amount of 0.1 to 5.0% by weight of the pouch composition, such as 0.1 to 3.0% by weight of the pouch composition, such as 0.2 to 2.0% by weight of the pouch composition, such as 0.3 to 1.5% by weight of the pouch composition, such as 0.4 to 1.0% by weight of the pouch composition.

According to an advantageous embodiment of the invention, the pouch composition comprises said alginate in a weight ratio of 1:2 to 3:1 relative to the amount of divalent cation, such as 1:1 to 2:1 relative to the amount of divalent cation.

In the above embodiment, the amount of the alginate refers to the alginate salt where appliable and the amount of divalent cation refers to the compound comprising divalent cation.

According to an advantageous embodiment of the invention, the pouch composition comprises said alginate in a weight ratio of 1:5 to 3:1 relative to the amount of phosphate, such as 1:3 to 1:1 relative to the amount of phosphate, such as 1:2 to 2:3 relative to the amount of phosphate.

According to an embodiment of the invention, the pouch composition comprises said alginate in a weight ratio of 1:2 to 3:1 relative to the amount of phosphate, such as 1:5 to 1:1 relative to the amount of phosphate, such as 1:3 to 2:3 relative to the amount of phosphate.

According to an embodiment of the invention, the pouch composition comprises said alginate in a weight ratio of 1:5 to 1:1 relative to the amount of phosphate, such as 1:3 to 2:3 relative to the amount of phosphate.

In the above embodiment, the amount of the alginate refers to the alginate salt where appliable and the amount of phosphate refers to the compound comprising phosphate.

According to an advantageous embodiment of the invention, the pouch composition comprises said divalent cations in a weight ratio of 1:8 to 1:1 relative to the amount of phosphate, such as 1:4 to 1:2 relative to the amount of phosphate.

In the above embodiment, the amount of the divalent cation refers to the compound comprising divalent cation and the amount of phosphate refers to the compound comprising phosphate.

According to an advantageous embodiment of the invention, the at least one pH regulating agent comprises at least one further pH regulating agent.

Thus, in the above embodiment, the pouch composition comprises phosphate and at least one further pH regulating agent.

According to an advantageous embodiment of the invention, the at least one further pH regulating comprises a buffering agent, such as an alkaline buffering agent.

In an embodiment of the invention, the at least one further pH regulating consist of a buffering agent.

According to an advantageous embodiment of the invention, the at least one further pH regulating agent is an alkaline pH regulating agent, such as an alkaline buffering agent.

An advantage of the above embodiment may be that a more efficient absorption of nicotine through the oral mucosa may be facilitated.

According to an advantageous embodiment of the invention, the at least one further pH regulating agent is selected from the group consisting of sodium carbonate, sodium bicarbonate, potassium carbonate, and magnesium carbonate, potassium bicarbonate, trometamol, or any combination thereof.

In the present context the term trometamol refers to (tris(hydroxymethyl)aminomethane), also sometimes referred to as tris buffer.

According to an embodiment of the invention, the pouch composition comprises said further pH regulating agent in an amount of at least 0.01% by weight of the pouch composition, such as in an amount of at least 0.5% by weight of the pouch composition, such as in an amount of at least 1% by weight of the pouch composition, such as in an amount of at least 2% by weight of the pouch composition, such as in an amount of at least 3% by weight of the pouch composition.

According to an embodiment of the invention, the pouch composition comprises said further pH regulating agent in an amount of no more than 15% by weight of the pouch composition, such as in an amount of no more than 10% by weight of the pouch composition, such as in an amount of no more than 8% by weight of the pouch composition.

According to an advantageous embodiment of the invention, the pouch composition comprises said further pH regulating agent in an amount of 0.01 to 15% by weight of the pouch composition, such as in an amount of 0.5 to 10% by weight of the pouch composition, such as in an amount of 1 to 10% by weight of the pouch composition, such as in an amount of 2 to 10% by weight of the pouch composition, such as in an amount of 3 to 8% by weight of the pouch composition.

In an embodiment of the invention, said further pH regulating agent is water-soluble.

In an embodiment of the invention, said further pH regulating agent has a water-solubility of at least 5 gram per 100 mL of water measured at 25 degrees Celsius, atmospheric pressure and pH 7.0.

In an embodiment of the invention, said further pH regulating agent has a water-solubility of at least 5 to 500 gram per 100 mL of water measured at 25 degrees Celsius, atmospheric pressure and pH 7.0.

An advantage of the above embodiment may be that a relatively effective uptake of nicotine is facilitated due to the high pH value obtained.

According to an advantageous embodiment of the invention, the nicotine is selected from the group consisting of a nicotine salt, nicotine free base, a nicotine-ion exchange resin combination, a nicotine inclusion complex or nicotine in any non-covalent binding; nicotine bound to zeolites, nicotine bound to cellulose, such as microcrystalline cellulose, starch microspheres, and mixtures thereof.

According to an advantageous embodiment of the invention, the nicotine comprises non-salt nicotine.

In an embodiment of the invention, the nicotine consists of non-salt nicotine.

According to an advantageous embodiment of the invention, the nicotine comprises nicotine free base.

In an embodiment of the invention, the nicotine comprises nicotine free base.

According to an advantageous embodiment of the invention, the nicotine comprises nicotine mixed with ion exchange resin.

In an embodiment of the invention, the nicotine comprises nicotine mixed with ion exchange resin.

According to an advantageous embodiment of the invention, the nicotine comprises free-base nicotine mixed with ion exchange resin in a weight ratio between the free-base nicotine and the ion exchange resin of 0.1 to 2.0, preferably from 0.5 to 2.0, and most preferred about 0.67 to 1.0.

Thus, in an embodiment of the invention, the nicotine comprises free-base nicotine mixed with ion exchange resin in a weight ratio between the free-base nicotine and the ion exchange resin of 1:10 to 2:1, preferably from 1:2 to 2:1, and most preferred about 2:3 to 1:1.

According to an advantageous embodiment of the invention, the nicotine comprises nicotine bound to an ion exchange resin.

In an embodiment of the invention, the nicotine comprises nicotine bound to an ion exchange resin.

In an embodiment of the invention, the ion exchange resin comprises one or more resin(s) selected from the group consisting of:
(i) a methacrylic, weakly acidic type of resin containing carboxylic functional groups,
(ii) a copolymer of methacrylic acid and divinylbenzene, said copolymer containing carboxylic functional groups,
(iii) a polystyrene, strongly acidic type of resin containing sulphonic functional groups,
(iv) a polystyrene, intermediate acidic type of resin containing phosphonic functional groups, and
(v) a combination thereof.

In an embodiment of the invention, the ion exchange resin comprises polacrilex resin.

In an embodiment of the invention, the ion exchange resin is polacrilex resin.

The term NPR may be used as an abbreviation to denote nicotine complexed with the ion-exchange resin specifically known as polacrilex resin.

According to an advantageous embodiment of the invention, the nicotine comprises a nicotine salt.

In an embodiment of the invention, the nicotine comprises a nicotine salt.

In an embodiment of the invention, the nicotine salt is selected from nicotine ascorbate, nicotine aspartate, nicotine benzoate, nicotine monotartrate, nicotine bitartrate, nicotine chloride (e.g., nicotine hydrochloride and nicotine dihydrochloride), nicotine citrate, nicotine fumarate, nicotine gensitate, nicotine lactate, nicotine mucate, nicotine laurate, nicotine levulinate, nicotine malate nicotine perchlorate, nicotine pyruvate, nicotine salicylate, nicotine sorbate, nicotine succinate, nicotine zinc chloride, nicotine sulfate, nicotine tosylate and hydrates thereof (e.g., nicotine zinc chloride monohydrate).

According to an advantageous embodiment of the invention, the nicotine salt comprises nicotine bitartrate.

The term NBT may be used as an abbreviation to denote nicotine bitartrate.

According to an embodiment of the invention, the nicotine salt consists of nicotine bitartrate.

According to an advantageous embodiment of the invention, the nicotine comprises synthetic nicotine.

In an embodiment of the invention, the nicotine comprises synthetic nicotine.

According to an advantageous embodiment of the invention, the pouch composition comprises nicotine in an amount of at least 0.1% by weight of the pouch composition, such as at least 0.25% by weight of the pouch composition, such as at least 0.5% by weight of the pouch composition, such as at least 1.0% by weight of the pouch composition, such as at least 2.0% by weight of the pouch compositions, such as at least 4.0% by weight of the pouch compositions.

In an embodiment of the invention, the pouch composition comprises nicotine in an amount of no more than 15.0% by weight of the pouch composition, such as no more than 10.0% by weight of the pouch composition, such as no more than 8.0% by weight of the pouch composition, such as no more than 5.0% by weight of the pouch compositions.

In an embodiment of the invention, the pouch composition comprises nicotine in an amount of 0.1% to 15.0% by weight of the pouch composition, such as 0.25% to 10.0% by weight of the pouch composition, such as 1.0% to 8.0% by weight of the pouch composition, such as 2.0% to 5.0% by weight of the pouch compositions.

According to an advantageous embodiment of the invention, the pouch composition comprises nicotine-ion exchange combination in an amount of 0.1 to 20% by weight of the pouch composition, such as 1 to 18% by weight of the pouch composition, such as 5-16% by weight of the pouch composition.

According to an advantageous embodiment of the invention, the nicotine-ion exchange resin combination comprises nicotine complexed with ion exchange resin, wherein the nicotine constitutes an amount of between 5 and 50% by weight of nicotine-ion exchange resin combination.

According to an advantageous embodiment of the invention, the nicotine-ion exchange resin combination comprises nicotine in an amount of between 5 and 50% by weight of the nicotine-ion exchange resin combination and ion-exchange resin in an amount between 10 and 95% by weight of the nicotine-ion exchange resin combination.

According to an advantageous embodiment of the invention, the pouch composition comprises water in an amount of at least 10% by weight of the pouch composition, such as at least 15% by weight of the pouch composition, such as at least 20% by weight of the pouch composition.

An advantage of the above embodiment may be that a desirable and soft texture of the pouched product is facilitated. Also, by including substantial amounts of water, release of nicotine may be facilitated. If the water content of the pouch composition is low, the pouch composition may absorb saliva for an undesirably long period, whereas a sufficiently wet pouch composition may facilitate fast release of nicotine.

An advantage of the above embodiment may be a relatively fast nicotine release.

An advantage of the above embodiment may be a more effective release of nicotine.

In one embodiment of the invention, the pouch composition comprises water in an amount of no more than 20% by weight of the pouch composition, such as no more than 15% by weight of the pouch composition, such as no more than 10% by weight of the pouch composition.

According to an advantageous embodiment of the invention, the pouch composition comprises water in an amount of no more than 65% by weight of the pouch composition, such as no more than 60% by weight of the pouch composition, such as no more than 50% by weight of the pouch composition, such as no more than 40% by weight of the pouch composition.

According to an advantageous embodiment of the invention, the pouch composition comprises water in an amount of 5-65% by weight of the pouch composition, such as 10-65% by weight of the pouch composition, such as 15-60% by weight of the pouch composition, such as 15-50% by weight of the pouch composition, such as 20-50% by weight of the pouch composition, such as 20-40% by weight of the pouch composition.

In an embodiment of the invention, the pouch composition comprises water in an amount of 15-65% by weight of the pouch composition, such as 20-65% by weight of the pouch composition, such as 25-65°% by weight of the pouch composition.

In an embodiment of the invention, the pouch composition comprises water in an amount of 15-65% by weight of the pouch composition, such as 15-60% by weight of the pouch composition, such as 15-50% by weight of the pouch composition, such as 15-40% by weight of the pouch composition.

In an embodiment of the invention, the pouch composition comprises water in an amount of 15-60% by weight of the pouch composition, such as 15-50% by weight of the pouch composition, such as 15-40% by weight of the pouch composition, such as 15-30% by weight of the pouch composition.

In an embodiment of the invention, the pouch composition comprises water in an amount of 15-40% by weight of the pouch composition.

According to an advantageous embodiment of the invention, the pouch composition comprises at least one sugar alcohol.

An advantage of the above embodiment may be that a desirable sweetness is provided, e.g. for support of the flavor profile, and while retaining an attractive texture.

According to an advantageous embodiment of the invention, said at least one sugar alcohol comprises one or more from the group consisting of xylitol, maltitol, mannitol, erythritol, isomalt, sorbitol, lactitol, glycerol, and any combinations thereof.

An advantage of the above embodiment may be that a desirable sweetness is provided, e.g. for support of the flavor profile, and while retaining an attractive texture.

Thus, in the above embodiment, the at least one sugar alcohol may include mixtures of different types of sugar alcohols, such as e.g. hydrogenated starch hydrolysates, which comprises a mixture of primarily maltitol, sorbitol and further sugar alcohols.

In an embodiment of the invention, said at least one sugar alcohol is selected from the group consisting of xylitol, maltitol, mannitol, erythritol, isomalt, sorbitol, lactitol, glycerol, and any combinations thereof.

According to an advantageous embodiment of the invention, said at least one sugar alcohol comprises one or more from the group consisting of xylitol, maltitol, mannitol, erythritol, isomalt, sorbitol, lactitol, and any combinations thereof.

In an embodiment of the invention, said at least one sugar alcohol is selected from the group consisting of xylitol, maltitol, mannitol, erythritol, isomalt, sorbitol, lactitol, and any combinations thereof.

In particular, by utilizing sugar alcohols with desirable characteristics with respect to e.g. melting point - e.g. being solid at ambient temperature of 25 degrees Celsius - a desirable texture is facilitated.

In an embodiment of the invention, the pouch composition comprises sugar alcohol selected from the group consisting of xylitol, maltitol, mannitol, erythritol, isomalt, sorbitol, lactitol, glycerol, and any combinations thereof in an amount of at least 1% by weight of the pouch composition, such as at least 2% by weight of the pouch composition, such as at least 5% by weight of the pouch composition, such as at least 10% by weight of the pouch composition, such as at least 15% by weight of the pouch composition.

In an embodiment of the invention, the pouch composition comprises sugar alcohol selected from the group consisting of xylitol, maltitol, mannitol, erythritol, isomalt, sorbitol, lactitol, glycerol, and any combinations thereof in an amount of 1 to 80% by weight of the pouch composition, such as 2 to 70% by weight of the pouch composition, such as 5 to 60% by weight of the pouch composition, such as 10 to 50% by weight of the pouch composition, such as 15 to 40% by weight of the pouch composition, such as 15 to 30% by weight of the pouch composition.

In an embodiment of the invention, the pouch composition comprises sugar alcohol selected from the group consisting of xylitol, maltitol, mannitol, erythritol, isomalt, sorbitol, lactitol, and any combinations thereof in an amount of at least 1% by weight of the pouch composition, such as at least 2% by weight of the pouch composition, such as at least 5% by weight of the pouch composition, such as at least 10% by weight of the pouch composition, such as at least 15% by weight of the pouch composition.

In an embodiment of the invention, the pouch composition comprises sugar alcohol selected from the group consisting of xylitol, maltitol, mannitol, erythritol, isomalt, sorbitol, lactitol, and any combinations thereof in an amount of 1 to 80% by weight of the pouch composition, such as 2 to 70% by weight of the pouch composition, such as 5 to 60% by weight of the pouch composition, such as 10 to 50% by weight of the pouch composition, such as 15 to 40% by weight of the pouch composition, such as 15 to 30% by weight of the pouch composition.

According to an advantageous embodiment of the invention, said at least one sugar alcohol comprises at least one non directly compressible (non-DC) grade sugar alcohol.

According to an advantageous embodiment of the invention, the pouch composition comprises said at least one sugar alcohol in an amount of at least 1% by weight of the pouch composition, such as at least 2% by weight of the pouch composition, such as at least 5% by weight of the pouch composition, such as at least 10% by weight of the pouch composition, such as at least 15% by weight of the pouch composition.

According to an advantageous embodiment of the invention, the pouch composition comprises said at least one sugar alcohol in an amount of 1 to 80% by weight of the pouch composition, such as 2 to 70% by weight of the pouch composition, such as 5 to 60% by weight of the pouch composition, such as 10 to 50% by weight of the pouch composition, such as 15 to 50% by weight of the pouch composition, such as 15 to 40% by weight of the pouch composition, such as 15 to 30% by weight of the pouch composition.

In an embodiment of the invention, the pouch composition comprises said at least one sugar alcohol in an amount of 5 to 70% by weight of the pouch composition, such as 10 to 70% by weight of the pouch composition, such as 15 to 70% by weight of the pouch composition, such as 20 to 70% by weight of the pouch composition, such as 20 to 60% by weight of the pouch composition, such as 20 to 50% by weight of the pouch composition, such as 20 to 40% by weight of the pouch composition.

According to an advantageous embodiment of the invention, the pouch composition comprises water-insoluble fiber.

In an embodiment of the invention, the water-insoluble fiber comprises a non-tobacco fiber.

According to an advantageous embodiment of the invention, the water-insoluble fiber is a plant fiber.

According to an advantageous embodiment of the invention, the water-insoluble fiber is a non-tobacco fiber.

According to an advantageous embodiment of the invention, the pouch composition comprises non-tobacco water-insoluble fiber in an amount of at least 5% by weight of the pouch composition, such as at least 10% by weight of the pouch composition, such as at least 15% by weight of the pouch composition, such as at least 20% by weight of the pouch composition.

Thus, the pouch composition of the above embodiment may comprise substantial amounts of non-tobacco water-insoluble fiber and in such cases may be a non-tobacco pouch composition or a low tobacco pouch composition.

In an embodiment of the invention, the pouch composition comprises non-tobacco water-insoluble fiber in an amount of no more than 60% by weight of the pouch composition, such as no more than 50% by weight of the pouch composition, such as no more than 40% by weight of the pouch composition, such as no more than 40% by weight of the pouch composition.

In an embodiment of the invention, the pouch composition comprises non-tobacco water-insoluble fiber in an amount of 5-60% by weight of the pouch composition, such as 10-50% by weight of the pouch composition, such as 15-40% by weight of the pouch composition, such as 20-40% by weight of the pouch composition.

According to an advantageous embodiment of the invention, the water-insoluble fibers are free of tobacco fibers.

Thus, in the above embodiment the water-insoluble fibers do not comprise any tobacco fibers or any fibers derived from tobacco, i.e. the water-insoluble fiber is a non-tobacco fiber. Whereas nicotine may be derived from tobacco or from other sources, the water-insoluble fibers do not comprise any tobacco fibers in the above embodiment.

According to an advantageous embodiment of the invention, the pouch composition is free of tobacco fibers.

Thus, in the above embodiment the pouch composition does not comprise any tobacco fibers or any fibers derived from tobacco. Of course, whereas the nicotine may be derived from tobacco or from other sources, the pouch composition does not comprise any tobacco fibers in the above embodiment.

According to an advantageous embodiment of the invention, the pouch composition comprises said water-insoluble fiber in an amount of at least 5% by weight of the pouch composition, such as at least 10% by weight of the pouch composition, such as at least 15% by weight of the pouch composition, such as at least 20% by weight of the pouch composition.

An advantage of the above embodiment may be that a desirable texture is facilitated e.g. by the ability of the water-insoluble fiber to hold substantial amounts of water.

According to an advantageous embodiment of the invention, the pouch composition comprises said water-insoluble fiber in an amount of no more than 70 % by weight of the pouch composition, such as no more than 60% by weight of the pouch composition, such as no more than 45% by weight of the pouch composition, such as no more than 40% by weight of the pouch composition.

According to an advantageous embodiment of the invention, the pouch composition comprises said water-insoluble fiber in an amount of 5 to 70 % by weight of the pouch composition, such as 5 to 60% by weight of the pouch composition, such as 10 to 45% by weight of the pouch composition, such as 15 to 40% by weight of the pouch composition, such as 20 to 40% by weight of the pouch composition.

In an embodiment of the invention, the pouch composition comprises said water-insoluble fiber in an amount of 5 to 50 % by weight of the pouch composition, such as 5 to 45% by weight of the pouch composition, such as 5 to 40% by weight of the pouch composition.

In an embodiment of the invention, the pouch composition comprises said water-insoluble fiber in an amount of 10 to 70% by weight of the pouch composition, such as 10 to 60% by weight of the pouch composition, such as 10 to 50% by weight of the pouch composition, such as 15 to 50% by weight of the pouch composition.

According to an advantageous embodiment of the invention, the water-insoluble fiber comprises one or more selected from the group consisting of wheat fibers, pea fibers, rice fiber, maize fibers, oat fibers, tomato fibers, barley fibers, rye fibers, sugar beet fibers, buckwheat fibers, potato fibers, apple fibers, cocoa fibers, bran fibers, bamboo fibers, powdered cellulose, and any combination thereof.

In an embodiment of the invention, the water-insoluble fiber is selected from the group consisting of wheat fibers, pea fibers, rice fiber, maize fibers, oat fibers, tomato fibers, barley fibers, rye fibers, sugar beet fibers, buckwheat fibers, potato fibers, apple fibers, cocoa fibers, bran fibers, bamboo fibers, powdered cellulose, and any combination thereof.

According to an advantageous embodiment of the invention, the water-insoluble fiber comprises microcrystalline cellulose.

According to an advantageous embodiment of the invention, the water-insoluble fiber is free of microcrystalline cellulose.

In an embodiment of the invention, the water-insoluble fiber has a water binding capacity of at least 200%, such as at least 300%, such as at least 400%.

In an embodiment of the invention, the water-insoluble fiber has a water binding capacity of 200% to 1500%, such as 300 to 1300%, such as 200 to 800%, such as 300 to 800%, such as 400 to 600%.

In an embodiment of the invention, the water-insoluble fiber has a density of 50 to 500 gram per Liter, such as 100 to 400 gram per Liter, such as 200 to 300 gram per Liter.

In an embodiment of the invention, the pouch composition comprises water-insoluble fiber selected from the group consisting of wheat fibers, pea fibers, rice fiber, maize fibers, oat fibers, tomato fibers, barley fibers, rye fibers, sugar beet fibers, buckwheat fibers, potato fibers, apple fibers, cocoa fibers, bran fibers, bamboo fibers, powdered cellulose, and any combination thereof in an amount of at least 5% by weight of the pouch composition, such as at least 10% by weight of the pouch composition, such as at least 15% by weight of the pouch composition.

According to an advantageous embodiment of the invention, the pouch composition comprises tobacco in an amount of no more than 5% by weight of the pouch composition, such as no more than 2% by weight of the pouch composition, such as no more than 1% by weight of the pouch composition, such as no more than 0.5% by weight of the pouch composition, such as no more than 0.1% by weight of the pouch composition.

In an embodiment of the invention, the pouch composition comprises tobacco in an amount of 0.001 - 5% by weight of the pouch composition, such as 0.01 - 5% by weight of the pouch composition, such as 0.05 - 2% by weight of the pouch composition, such as 0.1 - 1% by weight of the pouch composition, such as 0.2 - 0.5% by weight of the pouch composition.

According to an advantageous embodiment of the invention, the pouch composition comprises humectant.

To the extent that the alginate comprised in the pouch composition is considered a humectant, the above embodiment refers to a further humectant.

In an embodiment, the humectant is selected from the group consisting of glycerol, propylene glycol, pectin, modified starch, hydroxypropyl cellulose, triacetin, polyethylene glycol (PEG), xanthan gum, and combinations thereof.

In an embodiment of the invention, the humectant is selected from the group consisting of modified starch, triacetin, polyethylene glycol (PEG), pectin, and xanthan gum, or any combination thereof.

In an embodiment of the invention, the humectant is selected from the group consisting of propylene glycol, hydroxypropyl cellulose and glycerol, or any combination thereof.

An advantage of the above embodiment may be that a combination of a high nicotine release rate may be obtained together with a desirable mouthfeel, hereunder softness and moisture feeling.

A further advantage of the above embodiment may be that a desirable flavor perception may be obtained, facilitated by a combination of an improved flavor release and a desirable mouthfeel, hereunder softness and moisture feeling.

Advantages of the above embodiment may be improved release of nicotine, improved release of flavor, and improved flavor sensation without compromising the moisture mouth feel.

In an embodiment, the pouch composition comprises humectant in an amount of 0.1 to 5%, such as 0.2 to 5% by weight of the pouch composition, such as 0.5-2% by weight of the pouch composition.

According to an advantageous embodiment of the invention, the pouch composition comprises flavor.

In an embodiment of the invention, the pouch composition comprises flavor in an amount of at least 0.01% by weight of the pouch composition.

In an embodiment of the invention, the pouch composition comprises flavor in an amount of at least 15% by weight of the pouch composition.

In an embodiment of the invention, the pouch composition comprises flavor in an amount of 0.01 - 15% by weight of the pouch composition.

In an embodiment of the invention, the flavor comprises liquid flavor.

In an embodiment of the invention, the flavor comprises powdered flavor.

In an embodiment of the invention, the flavor comprises liquid flavor and powdered flavor.

According to an advantageous embodiment of the invention, the pouch composition is a powdered composition.

As used herein the term "powder composition" refers to composition in the form of powder, i.e. as a particulate material having a relatively small particle size, for example between 1 and 1200 micrometer. Particularly, by powder composition is not meant a powdered tobacco.

It is also disclosed an oral nicotine pouch composition comprising
nicotine,
water in an amount of at least 5% by weight of the pouch composition, alginate,
inorganic multivalent cations, and
at least one pH regulating agent comprising inorganic phosphate.

Said multivalent cations are selected from the group consisting of multivalent ions of calcium, magnesium, zinc, aluminum, barium, iron, manganese, copper, lead, cobalt, nickel, such as Ca2+, Mg2+, Zn2+, Al3+, Ba2+, Fe2+, Fe3+, Fe4+, Mn2+, Mn4+, Cu4+, and any combinations thereof.

The multivalent cations are trivalent.

The multivalent cations are selected from the group consisting of trivalent cations of aluminum, divalent cations of calcium, magnesium, iron, zinc, and any combination thereof.

The multivalent cations are selected from the group consisting of divalent cations of calcium, magnesium, iron, zinc, and any combination thereof.

The invention further relates to a nicotine pouched product comprising a pouch composition according to the invention or any of its embodiments and a saliva-permeable pouch enclosing said pouch composition.

According to an advantageous embodiment of the invention, the pouched product comprises said pouch composition in an amount of at least 50 mg, such as at least 100 mg, such as at least 150 mg, such as at least 200 mg, such as at least 300 mg.

In an embodiment of the invention, the pouched product comprises said pouch composition in an amount of no more than 700 mg, such as no more than 600 mg, such as no more than 500 mg, such as no more than 400 mg, such as no more than 350 mg, such as no more than 300 mg, such as no more than 250 mg, such as no more than 200 mg.

In an embodiment of the invention, the pouched product comprises said pouch composition in an amount of 50-700 mg, such as 60-600 mg, such as 75-500 mg, such as 75-400 mg, such as 75-350 mg, such as 100-300 mg, such as 125-250 mg, such as 150-200 mg.

In an embodiment of the invention, the pouched product comprises said pouch composition in an amount of 100-700 mg, such as 150-600 mg, such as 200-500 mg, such as 300-400 mg.

According to an advantageous embodiment of the invention, the pouched product comprises nicotine in an amount of at least 1 mg, such as at least 2 mg, such as at least 4 mg, such as at least 6 mg.

In an embodiment of the invention, the pouched product comprises nicotine in an amount of no more than 25 mg, such as no more than 20 mg, such as no more than 15 mg, such as no more than 10 mg.

In an embodiment of the invention, the pouched product comprises nicotine in an amount of 1 mg to 25 mg, such as 2 mg to 20 mg, such as 4 mg to 15 mg, such as 6 mg to 10 mg.

### DETAILED DESCRIPTION

As used herein the term "pouch composition" refers to the composition for use in an oral pouch, i.e. in pouches for oral use. Thus, pouch composition refers to the composition enclosed within the pouch. Also, the terms "pouch composition", "nicotine pouch composition" and "solid oral nicotine formulation" are used interchangeably, when referring to the composition being enclosed within the pouch.

As used herein the term "nicotine" refers to nicotine used as a refined/isolated substance. Particularly, nicotine does not refer to tobacco materials having a content of nicotine. Thus, when referring to nicotine amounts also to be understood as the nicotine dose, the amounts refer to the amount of pure nicotine.

Nicotine also covers nicotine not obtained from tobacco, often referred to as synthetic nicotine.

As used herein, unless specifically noted otherwise, a "ratio" refers to the weight ratio, i.e. the weight of a first component divided by the weight of a second component.

As used herein the term "free-base nicotine" refers to non-protonated form of nicotine, and therefore does not include nicotine salts or nicotine provided as a complex between nicotine and an ion exchange resin. Nevertheless, the free-base nicotine may be mixed with an amount of ion exchange resin or water-soluble compositions such as sugar alcohols or water-soluble fibers. While free-base nicotine includes both free-base nicotine extracted from tobacco as well as synthetically manufactured free-base nicotine, the free-base nicotine is not provided in the form of tobacco or powdered tobacco. Typically, free-base nicotine is provided as a liquid.

As used herein the term "pouch" is intended to mean a container typically formed by a web of a fibrous material enclosing a cavity. The pouch is pouch designed for administration of an active ingredient in the oral cavity, and thus it is adapted for oral use, it is non-toxic and not water-soluble. The fibrous material may e.g. form a woven or non-woven web or fabric. The pouch may for example be sealed by bonding two corresponding pieces of web or fabric to each other along their edges to form a cavity for the nicotine and the non-water-soluble composition. In order to release the nicotine, the pouch is made water-permeable so as to allow saliva from the oral cavity to penetrate the pouch and enter the cavity, where the saliva can come into contact with the nicotine, whereby the nicotine are released from the oral pouch.

As used herein, the term "nicotine-ion exchange resin combination" refer to a combination comprising nicotine complexed with ion exchange resin and/or free-base nicotine mixed with ion exchange resin.

As used herein, the term "nicotine complexed with ion-exchange resin" refers to nicotine bound to an ion exchange resin.

In the present context, the term "free-base nicotine mixed with ion exchange resin" refers to a mixture comprising free-base nicotine and ion exchange resin. It is noted that even if some embodiments comprise a combination of nicotine complexed with ion exchange resin and nicotine in its free-base form mixed with ion exchange resin, the term "free-base nicotine mixed with ion exchange resin" requires the presence of nicotine in its free-base form. In some embodiments, the mixture is an aqueous mixture. Free-base nicotine and water is mixed with ion-exchange resin, whereby a mixture comprising both free-base nicotine and ion exchange resin is obtained. Free-base nicotine mixed with ion exchange resin is referred to as "premix" in the examples.

As used herein the term "powder composition" refers to composition in the form of powder, i.e. as a particulate material having a relatively small particle size, for example between 1 and 1200 micrometer. Particularly, by powder composition is not meant a powdered tobacco.

As used herein the term "humectant" is understood as a moistening agent used to keep pouches moist, i.e. a humectant is added to the pouch composition with the purpose of keeping the pouch moist. Hence, the term humectant does not refer to substances added for other purposes, hereunder also hygroscopic substances added for other purposes, such as sugar alcohols, water-insoluble fibers and glycerol associated with ion-exchange resin in nicotine-ion exchange resin combinations, such as nicotine polacrilex. Examples of humectants include propylene glycol, hydroxypropyl cellulose, and glycerol. It is noted that when glycerol is included as a humectant, the glycerol is added as free glycerol and therefore liquid at room temperature. Further examples of humectants include triacetin, modified starch, pectin, xanthan gum, etc. The term humectant does not refer to sugar alcohols comprising 4 or more carbons. Also, the term humectant does not refer to fibers, such as water-insoluble fiber, such as wheat fibers, pea fibers, rice fiber, maize fibers, oat fibers, tomato fibers, barley fibers, rye fibers, sugar beet fibers, buckwheat fibers, potato fibers, cellulose fibers, apple fibers, cocoa fibers, cellulose fibers, bran fibers, bamboo fibers, powdered cellulose, and combinations thereof. Also, the term humectant does not include e.g. NaCl.

As used herein the term "water-soluble" refers to a relatively high water-solubility, for example a water-solubility of more than 5 gram of water-soluble composition or substance per 100 mL of water measured at 25 degrees Celsius, atmospheric pressure and pH of 7.0. When referring to a "soluble" composition or substance, water-soluble is meant, unless otherwise stated.

As used herein the term "water-insoluble" refers to relatively low water-solubility, for example a water-solubility of less than 0.1 gram of composition or substance per 100 mL of water measured at 25 degrees Celsius, atmospheric pressure and pH of 7.0. When referring to "insoluble", water-insoluble is meant unless otherwise stated. Therefore, compositions or substances having a water-solubility of between 0.1 and 5 gram per of composition or substance per 100 mL of water measured at 25 degrees Celsius, atmospheric pressure and pH of 7.0 are considered neither water-soluble nor water-insoluble, but having an intermediate water-solubility.

The pouches of the invention provide a nicotine release into the oral cavity. A release profile of nicotine may be obtained which both comprises a fast release period and a sustained release period.

As used herein the term "fast release" or "fast release period" may refer to the initial 2 minutes of the nicotine release profile, whereas the term "sustained release period refers" to the subsequent period of the release profile until end of experiment or end of use.

As used herein the term "fast release rate" refers to the released nicotine per minute within the initial 2 minutes.

As used herein the term "effective release" refers to the total release of nicotine over the release period of the experiment or the use period.

As used herein, the term "dissolve" is the process where a solid substance enters a solvent (such as oral saliva or water within the pouch) to yield a solution.

Typically, the pouches comprise openings, where the characteristic opening dimension is adapted to a characteristic dimension of the matrix composition so as to retain the matrix composition inside the pouch before use and/or to retain a part of the matrix composition, such as a water-insoluble composition, inside the pouch during use.

In order to obtain a pouch having suitable opening dimensions in view of the matrix composition to be used, the material for the pouch may be selected accordingly, e.g. comprising e.g. woven and/or non-woven fabric.

In other words, according to the various embodiments, the pouch forms a membrane allowing passage of saliva and prevents or inhibits passage of said matrix composition. The membrane of the pouch may be of any suitable material e.g. woven or non-woven fabric (e.g. cotton, fleece etc.), heat sealable non-woven cellulose or other polymeric materials such as a synthetic, semi-synthetic or natural polymeric material. An example of suitable pouch material is paper made of pulp and a small amount of wet strength agent. A material suitable for use must provide a semi-permeable membrane layer to prevent the powder or composition from leaving the bag or pouch during use. Suitable materials are also those that do not have a significant impact on the release of nicotine from the pouch.

The pouch composition is filled into pouches and is maintained in the pouch by a sealing. An ideal pouch is chemically and physically stable, it is pharmaceutically acceptable, it is insoluble in water, it is easy to fill with powder and seal, and it provides a semi-permeable membrane layer which prevent the powder from leaving the bag, but permit saliva and therein dissolved or sufficiently small suspended components from the pouch composition in the pouch, such as nicotine, to pass through said pouch.

The pouch may be placed in the oral cavity by the user. Saliva then enters into the pouch, and the nicotine and other components, which are soluble in saliva, start to dissolve and are transported with the saliva out of the pouch into the oral cavity, where the nicotine may be absorbed.

According to an embodiment of the invention, the pouch composition may further comprise one or more additives.

In an embodiment of the invention, said additives are selected from the group consisting of bile salts, citrates, cyclodextrins, detergents, fatty acids, labrasol, lecithins, phospholipids, synthetic and natural surfactants, nonionic surfactants, solvents, steroidal detergents, solubilization agents, pH regulating agents, mucolytic or mucus clearing agents, membrane penetration-enhancing agents, or any combination thereof. pH regulating agents include buffers.

As used herein, the term "pH regulating agent" refers to agents, which active adjust and regulates the pH value of the solution to which they have been added or are to be added. Thus, pH regulating agents may be acids and bases, including acidic buffering agents and alkaline buffering agents. On the other hand, pH regulating agents does not including substances and compositions that can only affect the pH by dilution. Furthermore, pH regulating agents does not include e.g. flavoring, fillers, etc.

In an embodiment of the invention, said further pH-regulating agents are selected from the group consisting of Acetic acid, Adipic acid, Citric acid, Fumaric acid, Glucono-δ-lactone, Gluconic acid, Lactic acid, Malic acid, Maleic acid, Tartaric acid, Succinic acid, Propionic acid, Ascorbic acid, Carbonic acid, Sodium carbonate, Sodium bicarbonate, Potasium carbonate, trometamol, amino acids, or any combination thereof.

According to various embodiments of the invention, one or more sugar alcohols may be included in the pouch as part of the pouch composition, e.g. as a carrier or part thereof, or as a sweetener. Suitable sugar alcohols include sugar alcohols selected from the group of sorbitol, erythritol, xylitol, lactitol, maltitol, mannitol, hydrogenated starch hydrolyzates, isomalt, or any combination thereof.

In an embodiment of the invention the pouch composition comprises high intensity sweetener.

Preferred high intensity sweeteners include, but are not limited to sucralose, aspartame, salts of acesulfame, such as acesulfame potassium, alitame, saccharin and its salts, cyclamic acid and its salts, glycyrrhizin, dihydrochalcones, thaumatin, monellin, stevioside and the like, alone or in combination.

In an embodiment of the invention, the pouch composition comprises bulk sweeteners including sugar and/or sugarless components.

In an embodiment of the invention, the pouch composition comprises bulk sweetener in the amount of 1.0 to about 80% by weight of the pouch composition, more typically constitute 5 to about 70% by weight of the pouch composition, and more commonly 10 to 60% by weight of the pouch composition or 10-50% by weight of the pouch composition. Bulk sweeteners may function both as a sweetener and also as a humectant. In some embodiments, inclusion of certain ingredients may limit the about amounts of bulk sweetener further.

The sweeteners may often support the flavor profile of the pouch composition.

Sugar sweeteners generally include, but are not limited to saccharide-containing components commonly known in the art of pouches, such as sucrose, dextrose, maltose, saccharose, lactose, sorbose, dextrin, trehalose, D-tagatose, dried invert sugar, fructose, levulose, galactose, corn syrup solids, glucose syrup, hydrogenated glucose syrup, and the like, alone or in combination.

The sweetener can be used in combination with sugarless sweeteners. Generally, sugarless sweeteners include components with sweetening characteristics but which are devoid of the commonly known sugars and comprise, but are not limited to, sugar alcohols, such as sorbitol, mannitol, xylitol, hydrogenated starch hydrolyzates, maltitol, isomalt, erythritol, lactitol and the like, alone or in combination.

As used herein the term "flavor" is understood as having its ordinary meaning within the art. Flavor includes liquid and powdered flavors. Thus, flavors do of course not include sweeteners (such as sugar, sugar alcohols and high intensity sweeteners), or acids providing pure acidity/sourness, nor compounds providing pure saltiness (e.g. NaCl) or pure bitterness. Flavor enhancers include substances that only provide saltiness, bitterness or sourness. Flavor enhancers thus include e.g. NaCl, Citric acid, ammonium chloride etc. The flavors can be natural or synthetic flavors.

In an embodiment of the invention the pouch composition comprises flavor. Flavor may typically be present in amounts between 0.01 and 15% by weight of the total composition of the pouch, such as between 0.01 and 5% by weight of the total composition.

Non-exhaustive examples of flavors suitable in embodiments of the present invention are coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits mentioned above.

In various embodiments of the invention, the pouch composition comprises composition modifier. The composition modifier may be added to engineer the properties of the pouch composition and/or parts thereof, such as flowability, texture, homogeneity etc.

The composition modifiers may, according to various embodiments, be selected group consisting of metallic stearates, modified calcium carbonate, hydrogenated vegetable oils, partially hydrogenated vegetable oils, polyethylene glycols, polyoxyethylene monostearates, animal fats, silicates, silicates dioxide, talc, magnesium stearates, calcium stearates, fumed silica, powdered hydrogenated cottonseed oils, hydrogenated vegetable oils, hydrogenated soya oil, emulsifiers, triglycerides, and mixtures thereof. Particularly, metallic stearates, such as magnesium stearate, may be advantageous.

The composition modifiers may be added to the pouch composition in various ways.

For example, the composition modifiers may be added by full powder mixture during the last few minutes of the final mixing.

Alternatively, the composition modifiers may be added after granulation steps on a granulation premix.

The composition modifier, such as magnesium stearate, may have a sealing effect and can be used to control the release of the nicotine and the solubility of the pouch.

According to an embodiment of the invention, the pouch composition comprises polyvinylpyrrolidone (PVP). The pouch composition may also be free of PVP.

One advantage of the above embodiment may be that a more uniform composition may be obtained.

### EXAMPLES

### Example 1A - Preparation of pouches designed for administration of nicotine

The material of the pouches is heat sealable non-woven cellulose, such as long fiber paper. Pouches that are not in form of non-woven cellulose fabric may also be used according to the invention.

The powder is filled into pouches and is maintained in the pouch by a sealing.

### Example 1B - Preparation of pouches designed for administration of nicotine

The material of the pouches is manufactured using rayon fibers, such as viscose rayon staple fibers. The pouch membrane is heat sealed along its edges except for an opening in one end into an inner cavity formed by the pouch membrane.

The powder is filled into pouches and is maintained in the pouch by a sealing.

### Example 2: Preparation of nicotine premixes

A 60 liter planetary Bear Varimixer mixer was charged with water, and nicotine was weighed and added. The mixer was stirred at low speed for 1 minute at ambient temperature. Then ion exchange resin Amberlite ^{®} IRP64 was weighed and added to the mixer. The mixer was closed, stirred at high speed for 5 minutes, opened and scraped down, if necessary. Finally the mixer was stirred for further 5 minutes at high speed. The total process time was 20 minutes.

Thereby, mixtures of nicotine and cation exchange resin were produced from the constituents stated in the below tables.

### Premix I:

**Table 1. Ingredients used to manufacture nicotine premix I (5.7% nicotine). % water in obtained nicotine-resin composition: 71.4**

| Constituent | Amount (kg) | Amount (%) |
|---|---|---|
| Nicotine | 1.0 | 5.7 |
| Water | 12.5 | 71.4 |
| Resin | 4.0 | 22.9 |
| Total | 17.5 | 100.0 |

### Premix II:

**Table 2. Ingredients used to manufacture nicotine premix II (13.2% nicotine). % water in obtained nicotine-resin composition: 34.1.**

| Constituent | Amount (kg) | Amount (%) |
|---|---|---|
| Nicotine | 1.08 | 13.2 |
| Water | 2.80 | 34.1 |
| Resin | 4.32 | 52.7 |
| Total | 8.20 | 100.0 |

### Premix III:

**Table 3. Ingredients used to manufacture nicotine premix III (18.5% nicotine). % water in obtained nicotine-resin composition:7.5.**

| Constituent | Amount (kg) | Amount (%) |
|---|---|---|
| Nicotine | 1.08 | 18.5 |
| Water | 0.44 | 7.5 |
| Resin | 4.32 | 74.0 |
| Total | 5.84 | 100.0 |

### Premix IV:

**Table 4. Ingredients used to manufacture nicotine premix IV (10% nicotine). % water in obtained nicotine-resin composition: 50.0.**

| Constituent | Amount (kg) | Amount (%) |
|---|---|---|
| Nicotine | 1.08 | 10.0 |
| Water | 5.40 | 50.0 |
| Resin | 4.32 | 40.0 |
| Total | 10.8 | 100.0 |

### Premix V:

**Table 5. Ingredients used to manufacture nicotine premix V (20% nicotine). % water in obtained nicotine-resin composition: 31.5.**

| Constituent | Amount (kg) | Amount (%) |
|---|---|---|
| Nicotine | 1.78 | 20.0 |
| Water | 2.80 | 31.5 |
| Resin | 4.32 | 48.5 |
| Total | 8.90 | 100.0 |

### Premix VI:

**Table 6. Ingredients used to manufacture nicotine premix VI (30% nicotine). % water in obtained nicotine-resin composition: 27.5.**

| Constituent | Amount (kg) | Amount (%) |
|---|---|---|
| Nicotine | 3.05 | 30.0 |
| Water | 2.80 | 27.5 |
| Resin | 4.32 | 42.5 |
| Total | 10.17 | 100.0 |

### Premix VII

**Table 7. Ingredients used to manufacture nicotine premix VII (35% nicotine). % water in obtained nicotine-resin composition: 25.6.**

| Constituent | Amount (kg) | Amount (%) |
|---|---|---|
| Nicotine | 3.83 | 35.0 |
| Water | 2.80 | 25.6 |
| Resin | 4.32 | 39.4 |
| Total | 10.95 | 100.0 |

### Premix VIII:

**Table 8. Ingredients used to manufacture nicotine premix VIII (42% nicotine).. % water in obtained nicotine-resin composition: 22.8.**

| Constituent | Amount (kg) | Amount (%) |
|---|---|---|
| Nicotine | 5.15 | 42.0 |
| Water | 2.80 | 22.8 |
| Resin | 4.32 | 35.2 |
| Total | 12.27 | 100.0 |

### Example 3: Preparation of pouch compositions

Pouches are prepared comprising powdered compositions as outlined in table 9 - 21. The pouches are made as follows.

Fibers and water are mixed using a planetary Bear Varimixer mixer for 5 minutes. Then, the following ingredients were added subsequently under continuous mixing: first the nicotine (mixed for 2 minutes), then the remaining ingredients except liquid flavor and glidant if any (mixed for 2 minutes), then liquid flavor if any (mixed for 1 minute), then glidant if any (mixed for 1 minute). The total mixing time is 9-11 minutes.

For pouch compositions comprising no or low amounts of water, the pouch compositions may alternatively be made as follows.

Fibers and other dry ingredients are mixed using a planetary Bear Varimixer mixer for 5 minutes. Then, the following ingredients were added subsequently under continuous mixing: first the nicotine (mixed for 2 minutes), then the remaining liquid ingredients if any, except liquid flavor and glidant if any (mixed for 2 minutes), then liquid flavor if any (mixed for 1 minute), then glidant if any (mixed for 1 minute). The total mixing time is 9-11 minutes.

### Example 4: Preparation of filled pouches

The final pouch composition is filled into pouches (target fill weight 500 mg powder per pouch). The pouch material of example 1A or 1B may be used. The powder is filled into pouches and is maintained in the pouch by a sealing.

### Example 5A: Pouches

The pouch compositions are prepared from the ingredients in table 9 using preparation method described in example 3.

The pouch compositions are filled into pouches as described in example 4 (pouch material of examples 1A was used, but 1B could also have been applied).

**Table 9: Pouch compositions.**

| **Pouches** | **PAM 01** | **PAM 02** | **PAM 03** | **PAM 04** | **PAM 05** | **PAM 06** | **C1** |
|---|---|---|---|---|---|---|---|
| Amount of nicotine | 9.6 mg | 9.6 mg | 9.6 mg | 9.6 mg | 9.6 mg | 9.6 mg | 9.6 mg |
| Raw material | Content in weight percent | | | | | | |
| Premix II | 14.6 | 14.6 | 14.6 | 14.6 | 14.6 | 14.6 | 14.6 |
| CaSO4 | 0.1 | 0.5 | 2.5 | 0.2 | 1.0 | 2.5 | - |
| Xylitol | 16.8 | 14.9 | 13.4 | 16.6 | 13.4 | 7.4 | 13.4 |
| Purified water | 25 | 25 | 25 | 25 | 25 | 25 | 25 |
| Wheat fiber | 30 | 30 | 30 | 30 | 30 | 30 | 30 |
| Sodium alginate | 0.5 | 0.5 | 0.5 | 0.2 | 1.0 | 2.5 | 2.0 |
| Phosphate buffer | 1.0 | 1.0 | 1.0 | 0.4 | 2.0 | 5.0 | - |
| Sodium carbonate | 3.0 | 3.0 | 3.0 | 3.0 | 3.0 | 3.0 | 5.0 |
| Flavor | 6.9 | 6.9 | 6.9 | 6.9 | 6.9 | 6.9 | 6.9 |
| High intensity sweetener | 1.0 | 1.0 | 1.0 | 1.0 | 1.0 | 1.0 | 1.0 |
| Potassium sorbate | 0.1 | 0.1 | 0.1 | 0.1 | 0.1 | 0.1 | 0.1 |
| Silicon dioxide | 2.0 | 2.0 | 2.0 | 2.0 | 2.0 | 2.0 | 2.0 |
| Total | 100 | 100 | 100 | 100 | 100 | 100 | 100 |

| | | | | | | | |
|---|---|---|---|---|---|---|---|
| Pouch content: 500 mg total. | | | | | | | |

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, apple fibers, cocoa fibers, powdered cellulose, bran fibers, and bamboo fibers.

Sodium carbonate is used as an alkaline buffering agent together with phosphate buffer. Other buffering agents as described herein may also be used in combination with sodium carbonate or an alternative.

Phosphate buffer may be composed of a single phosphate, such as e.g. sodium phosphate, potassium phosphate, sodium pyrophosphate, sodium polyphosphate or of a combination of two or more phosphates, such as e.g. sodium phosphate and sodium pyrophosphate.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5B: Pouches

**Table 10: Pouch compositions.**

| **Pouches** | **PAM10** | **PAM11** | **PAM12** | **PAM13** | **PAM14** | **PAM15** |
|---|---|---|---|---|---|---|
| Amount of nicotine | 9.6 mg | 9.6 mg | 9.6 mg | 9.6 mg | 9.6 mg | 9.6 mg |
| Raw material | Content in weight percent | | | | | |
| Premix II | 14.6 | 14.6 | 14.6 | 14.6 | 14.6 | 14.6 |
| CaSO4 | 0.5 | 0.5 | 0.5 | 0.5 | 0.5 | 0.5 |
| Xylitol | 15.8 | 14.9 | 13.4 | 16.3 | 14.4 | 11.4 |
| Purified water | 25 | 25 | 25 | 25 | 25 | 25 |
| Wheat fiber | 30 | 30 | 30 | 30 | 30 | 30 |
| Sodium alginate | 0.1 | 1.0 | 2.5 | 0.5 | 0.5 | 0.5 |
| Phosphate buffer | 1.0 | 1.0 | 1.0 | 0.1 | 2.0 | 5.0 |
| Sodium carbonate | 3.0 | 3.0 | 3.0 | 3.0 | 3.0 | 3.0 |
| Flavor | 6.9 | 6.9 | 6.9 | 6.9 | 6.9 | 6.9 |
| High intensity sweetener | 1.0 | 1.0 | 1.0 | 1.0 | 1.0 | 1.0 |
| Potassium sorbate | 0.1 | 0.1 | 0.1 | 0.1 | 0.1 | 0.1 |
| Silicon dioxide | 2.0 | 2.0 | 2.0 | 2.0 | 2.0 | 2.0 |
| Total | 100 | 100 | 100 | 100 | 100 | 100 |

| | | | | | | |
|---|---|---|---|---|---|---|
| Pouch content: 500 mg total. | | | | | | |

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, apple fibers, cocoa fibers, powdered cellulose, bran fibers, and bamboo fibers.

Sodium carbonate is used as an alkaline buffering agent together with phosphate buffer. Other buffering agents as described herein may also be used in combination with sodium carbonate or an alternative.

Phosphate buffer may be composed of a single phosphate, such as e.g. sodium phosphate, potassium phosphate, sodium pyrophosphate, sodium polyphosphate or of a combination of two or more phosphates, such as e.g. sodium phosphate and sodium pyrophosphate.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5C: Pouches

The pouch compositions are prepared from the ingredients in table 11 using preparation method described in example 3.

The pouch compositions are filled into pouches as described in example 4 (pouch material of examples 1A was used, but 1B could also have been applied).

**Table 11: Pouch compositions.**

| **Pouches** | **PAM 20** | **PAM 21** | **PAM 22** | **PAM 23** | **PAM 24** | **PAM 25** |
|---|---|---|---|---|---|---|
| Amount of nicotine | 9.6 mg | 9.6 mg | 9.6 mg | 9.6 mg | 9.6 mg | 9.6 mg |
| Raw material | Content in weight percent | | | | | |
| Premix II | 14.6 | 14.6 | 14.6 | 14.6 | 14.6 | 14.6 |
| CaSO4 | 0.5 | 0.5 | 0.5 | 0.5 | 0.5 | 0.5 |
| Erythritol | 15.4 | - | - | - | 5.4 | - |
| Maltitol | - | 15.4 | - | - | - | - |
| Sorbitol | - | - | 15.4 | - | - | - |
| Xylitol | - | - | - | 5.2 | 11 | 32 |
| Purified water | 25 | 25 | 25 | 29.8 | 24.5 | 17.5 |
| Wheat fiber | 30 | 30 | 30 | 35.4 | 29.5 | 20.9 |
| Sodium alginate | 0.5 | 0.5 | 0.5 | 0.5 | 0.5 | 0.5 |
| Phosphate buffer | 1.0 | 1.0 | 1.0 | 1.0 | 1.0 | 1.0 |
| Sodium carbonate | 3.0 | 3.0 | 3.0 | 3.0 | 3.0 | 3.0 |
| Flavor | 6.9 | 6.9 | 6.9 | 6.9 | 6.9 | 6.9 |
| High intensity sweetener | 1.0 | 1.0 | 1.0 | 1.0 | 1.0 | 1.0 |
| Potassium sorbate | 0.1 | 0.1 | 0.1 | 0.1 | 0.1 | 0.1 |
| Silicon dioxide | 2.0 | 2.0 | 2.0 | 2.0 | 2.0 | 2.0 |
| Total | 100 | 100 | 100 | 100 | 100 | 100 |

| | | | | | | |
|---|---|---|---|---|---|---|
| Pouch content: 500 mg total. | | | | | | |

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, apple fibers, cocoa fibers, powdered cellulose, bran fibers, and bamboo fibers.

Sodium carbonate is used as an alkaline buffering agent together with phosphate buffer. Other buffering agents as described herein may also be used in combination with sodium carbonate or an alternative.

Phosphate buffer may be composed of a single phosphate, such as e.g. sodium phosphate, potassium phosphate, sodium pyrophosphate, sodium polyphosphate or of a combination of two or more phosphates, such as e.g. sodium phosphate and sodium pyrophosphate.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5D: Pouches

The pouch compositions are prepared from the ingredients in table 12 using preparation method described in example 3.

The pouch compositions are filled into pouches as described in example 4 (pouch material of examples 1A was used, but 1B could also have been applied).

**Table 12: Pouch compositions.**

| **Pouches** | **PAM 30** | **PAM 31** | **PAM 32** | **PAM 33** | **PAM 34** | **PAM 35** | **PAM 36** |
|---|---|---|---|---|---|---|---|
| Amount of nicotine | 9.6 mg | 9.6 mg | 9.6 mg | 9.6 mg | 9.6 mg | 9.6 mg | 9.6 mg |
| Raw material | Content in weight percent | | | | | | |
| Premix II | 14.6 | 14.6 | 14.6 | 14.6 | 14.6 | 14.6 | 14.6 |
| MgSO4 | - | - | - | 0.5 | - | - | - |
| CaCl2 | - | - | - | - | 0.5 | - | 0.5 |
| MgBr2 | - | - | - | - | - | 0.5 | - |
| CaSO4 | 0.5 | 0.5 | 0.5 | - | - | - | 0.5 |
| Xylitol | 18.4 | 16.9 | 14.4 | 15.4 | 15.4 | 15.4 | 14.9 |
| Purified water | 25 | 25 | 25 | 25 | 25 | 25 | 25 |
| Wheat fiber | 30 | 30 | 30 | 30 | 30 | 30 | 30 |
| Sodium alginate | 0.5 | 0.5 | 0.5 | 0.5 | 0.5 | 0.5 | 0.5 |
| Phosphate buffer | 1.0 | 1.0 | 1.0 | 1.0 | 1.0 | 1.0 | 1.0 |
| Tris | - | - | 4.0 | - | - | - | - |
| Sodium carbonate | - | 1.5 | - | 3.0 | 3.0 | 3.0 | 3.0 |
| Flavor | 6.9 | 6.9 | 6.9 | 6.9 | 6.9 | 6.9 | 6.9 |
| High intensity sweetener | 1.0 | 1.0 | 1.0 | 1.0 | 1.0 | 1.0 | 1.0 |
| Potassium sorbate | 0.1 | 0.1 | 0.1 | 0.1 | 0.1 | 0.1 | 0.1 |
| Silicon dioxide | 2.0 | 2.0 | 2.0 | 2.0 | 2.0 | 2.0 | 2.0 |
| Total | 100 | 100 | 100 | 100 | 100 | 100 | 100 |

| | | | | | | | |
|---|---|---|---|---|---|---|---|
| Pouch content: 500 mg total. | | | | | | | |

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, apple fibers, cocoa fibers, powdered cellulose, bran fibers, and bamboo fibers.

Sodium carbonate is used as an alkaline buffering agent together with phosphate buffer. Other buffering agents as described herein may also be used in combination with sodium carbonate or an alternative.

Phosphate buffer may be composed of a single phosphate, such as e.g. sodium phosphate, potassium phosphate, sodium pyrophosphate, sodium polyphosphate or of a combination of two or more phosphates, such as e.g. sodium phosphate and sodium pyrophosphate.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5E: Pouches

The pouch compositions are prepared from the ingredients in table 13 using preparation method described in example 3.

The pouch compositions are filled into pouches as described in example 4 (pouch material of examples 1A was used, but 1B could also have been applied).

**Table 13: Pouch compositions.**

| **Pouches** | **PAM 41** | **PAM 42** | **PAM 43** | **PAM 44** | **PAM 45** | **PAM 46** |
|---|---|---|---|---|---|---|
| Amount of nicotine | 9.6 mg | 9.6 mg | 9.6 mg | 9.6 mg | 4.8 | 14.7 |
| Raw material | Content in weight percent | | | | | |
| NPR(16%) | 12 | - | - | - | - | - |
| NBT | - | 5.5 | 1.8 | - | - | - |
| Premix VI | - | - | - | 4.0 | 3.2 | 5.8 |
| Premix II | - | - | 10 | 5.5 | | 9.1 |
| CaSO4 | 0.5 | 0.5 | 0.5 | 0.5 | 0.5 | 0.5 |
| Xylitol | 18 | 24.5 | 18.2 | 20.5 | 26.8 | 15.1 |
| Purified water | 25 | 25 | 25 | 25 | 25 | 25 |
| Wheat fiber | 30 | 30 | 30 | 30 | 30 | 30 |
| Sodium alginate | 0.5 | 0.5 | 0.5 | 0.5 | 0.5 | 0.5 |
| Phosphate buffer | 1.0 | 1.0 | 1.0 | 1.0 | 1.0 | 1.0 |
| Sodium carbonate | 3.0 | 3.0 | 3.0 | 3.0 | 3.0 | 3.0 |
| Flavor | 6.9 | 6.9 | 6.9 | 6.9 | 6.9 | 6.9 |
| High intensity sweetener | 1.0 | 1.0 | 1.0 | 1.0 | 1.0 | 1.0 |
| Potassium sorbate | 0.1 | 0.1 | 0.1 | 0.1 | 0.1 | 0.1 |
| Silicon dioxide | 2.0 | 2.0 | 2.0 | 2.0 | 2.0 | 2.0 |
| Total | 100 | 100 | 100 | 100 | 100 | 100 |

Pouch content: 500 mg total.

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, apple fibers, cocoa fibers, powdered cellulose, bran fibers, and bamboo fibers.

Sodium carbonate is used as an alkaline buffering agent together with phosphate buffer. Other buffering agents as described herein may also be used in combination with sodium carbonate or an alternative.

Phosphate buffer may be composed of a single phosphate, such as e.g. sodium phosphate, potassium phosphate, sodium pyrophosphate, sodium polyphosphate or of a combination of two or more phosphates, such as e.g. sodium phosphate and sodium pyrophosphate.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5F: Pouches

The pouch compositions are prepared from the ingredients in table 14A using preparation method described in example 3.

The pouch compositions are filled into pouches as described in example 4 (pouch material of examples 1A was used, but 1B could also have been applied).

**Table 14A: Pouch compositions.**

| **Pouches** | **PAM51** | **PAM52** | **PAM53** | **PAM54** |
|---|---|---|---|---|
| Amount of nicotine | 9.6 mg | 9.6 mg | 9.6 mg | 9.6 mg |
| Raw material | Content in weight percent | | | |
| Premix II | 14.6 | 14.6 | 14.6 | 14.6 |
| CaSO4 | 0.5 | 0.5 | 0.5 | 0.5 |
| Xylitol | 43.4 | 5.4 | 15.4 | 15.4 |
| Purified water | 12 | 29 | 25 | 25 |
| Oat fiber | 15 | 36 | - | - |
| Powdered cellulose | - | - | 30 | - |
| MCC | - | - | - | 30 |
| Sodium alginate | 0.5 | 0.5 | 0.5 | 0.5 |
| Phosphate buffer | 1.0 | 1.0 | 1.0 | 1.0 |
| Sodium carbonate | 3.0 | 3.0 | 3.0 | 3.0 |
| Flavor | 6.9 | 6.9 | 6.9 | 6.9 |
| High intensity sweetener | 1.0 | 1.0 | 1.0 | 1.0 |
| Potassium sorbate | 0.1 | 0.1 | 0.1 | 0.1 |
| Silicon dioxide | 2.0 | 2.0 | 2.0 | 2.0 |
| Total | 100 | 100 | 100 | 100 |

| | | | | |
|---|---|---|---|---|
| Pouch content: 500 mg total. Powdered cellulose, trade name "Vitacel L700G". Oat fiber, trade name "Vitacel HF 600". Microcrystalline cellulose (MCC), trade name "Vivapur 102". | | | | |

Other fibers may be used as well, such as water-insoluble plant fibers, such as wheat fibers, oat fibers, pea fibers, rice fiber, maize fibers, oat fibers, tomato fibers, barley fibers, rye fibers, sugar beet fibers, buckwheat fibers, potato fibers, apple fibers, cocoa fibers, powdered cellulose, bran fibers, and bamboo fibers.

Sodium carbonate is used as an alkaline buffering agent together with phosphate buffer. Other buffering agents as described herein may also be used in combination with sodium carbonate or an alternative.

Phosphate buffer may be composed of a single phosphate, such as e.g. sodium phosphate, potassium phosphate, sodium pyrophosphate, sodium polyphosphate or of a combination of two or more phosphates, such as e.g. sodium phosphate and sodium pyrophosphate.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 5G: Pouches

The pouch compositions are prepared from the ingredients in table 14B using preparation method described in example 3.

The pouch compositions are filled into pouches as described in example 4 (pouch material of examples 1A was used, but 1B could also have been applied).

**Table 14B: Pouch compositions.**

| **Pouches** | **C2** | **PAM 61** | **C3** | **PAM 62** | **C4** | **PAM 63** |
|---|---|---|---|---|---|---|
| Amount of nicotine | 7.5 mg | 7.5 mg | 7.5 mg | 7.5 mg | 10.0 mg | 10.0 mg |
| Raw material | Content in weight percent | | | | | |
| Premix II | 11.4 | 11.4 | 11.4 | 11.4 | 15.2 | 15.2 |
| CaSO4 | - | 0.2 | - | 0.2 | - | 0.3 |
| Erythritol | 16.0 | 16.0 | 17.0 | 17.0 | 13.6 | 13.6 |
| Purified water | 25 | 25 | 26 | 26 | 25 | 25 |
| Wheat fiber | 30 | 30 | 30 | 30 | 30 | 30 |
| Sodium alginate | 1.0 | 0.3 | 1.0 | 0.3 | 1.5 | 0.5 |
| Disodium phosphate (phosphate buffer) | - | 0.2 | - | 0.2 | - | 0.3 |
| Tetrasodium diphosphate (phosphate buffer) | - | 0.3 | - | 0.3 | - | 0.4 |
| Sodium carbonate | 3.0 | 3.0 | 3.0 | 3.0 | 3.0 | 3.0 |
| Flavor | 10.7 | 10.7 | 8.7 | 8.7 | 8.8 | 8.8 |
| High intensity sweetener | 0.8 | 0.8 | 0.8 | 0.8 | 0.8 | 0.8 |
| Potassium sorbate | 0.1 | 0.1 | 0.1 | 0.1 | 0.1 | 0.1 |
| Silicon dioxide | 2.0 | 2.0 | 2.0 | 2.0 | 2.0 | 2.0 |
| Total | 100 | 100 | 100 | 100 | 100 | 100 |

| | | | | | | |
|---|---|---|---|---|---|---|
| Pouch content: 500 mg total. | | | | | | |

Wheat fiber, trade name "Vitacel 600 WF plus". Other fibers may be used as well, such as water-insoluble plant fibers, such as oat fibers, pea fibers, rice fiber, maize fibers, oat fibers, tomato fibers, barley fibers, rye fibers, sugar beet fibers, buckwheat fibers, potato fibers, apple fibers, cocoa fibers, powdered cellulose, bran fibers, and bamboo fibers.

Sodium carbonate is used as an alkaline buffering agent together with phosphate buffer. Other buffering agents as described herein may also be used in combination with sodium carbonate or an alternative.

Phosphate buffer (if present) may be composed of a single phosphate, such as e.g. sodium phosphate, potassium phosphate, sodium pyrophosphate, sodium polyphosphate or of a combination of two or more phosphates, such as e.g. sodium phosphate and sodium pyrophosphate.

Flavor example, a mixture of e.g. menthol and peppermint may be used. Of course, other flavors as described herein may be use as well, in combination with menthol and/or peppermint or replacing these. The flavor may be liquid or flavored or a combination, i.e. a liquid flavor and a powdered flavor is added.

Acesulfame potassium and/or sucralose may as an example be used as high intensity sweeteners. Other usable high intensity sweeteners described herein may be used in combination with or instead of acesulfame potassium and/or sucralose.

Potassium sorbate is used as a preservative. Other preservatives as described herein may also be used in combination with or instead of potassium sorbate.

Silicon dioxide is used as a glidant. Other possible glidants include e.g. magnesium stearate, starch and talc.

### Example 6A: User evaluation.

The produced pouches of the invention were evaluated and found highly suitable as delivery vehicles of nicotine in that they provide a favorable release of nicotine and at the same time are pleasant to the user, e.g. with respect to a desirable mouthfeel such as a moist and moldable texture and a desirable taste.

### Example 6B: Processability

Pouch products were evaluated with respect to processability by evaluation of lump formation during processing.

Processability was further assessed by means of a sieving analysis using a sieving tower. Here, the product powder fractions passing certain sieve sizes during a fixed sieving time were measured. A higher powder fraction passing the sieve during the specified, fixed sieving time indicates an improved processability of the product powder.

### Example 7: Processability assessment

Pouch products PAM02 and C1 were evaluated with respect to processability by means of a sieving analysis using a sieving tower.

The analyses were performed on a Retsch AS 200 control sieve shaker with a sieve of mesh size 1.7 mm. The procedure for the sieving was as follows: A sample of 100 g product powder was placed on top of the sieve, the lid was fastened, and the shaker was started. The sample was shaken for 1 min with an amplitude of 0.6 mm.

The product powder fractions (weight %) passing the sieve after 1 min sieving time were measured, yielding the results as seen in Table 15.

**Table 15. Sieving tower analysis.**

| Sample ID | Powder fraction passing the sieve, wt % |
|---|---|
| C1 (comparative) | 38.4 |
| PAM02 | 69.4 |

PAM02 was found to have an excellent processability compared to the comparative sample as evident by the sieving test results.

### Example 8: Processability assessment

Pouch products C2, PAM61, C3, PAM62, C4 and PAM63 were evaluated with respect to processability by means of a sieving analysis using a sieving tower.

The analyses were performed on a Retsch AS 200 control sieve shaker with a sieve of mesh size 1.7 mm. The procedure for the sieving was as follows: A sample of 100 g product powder was placed on top of the sieve, the lid was fastened, and the shaker was started. The sample was shaken for 1 min with an amplitude of 0.6 mm.

The product powder fractions (weight %) passing the sieve after 1 min sieving time were measured, yielding the results as seen in Table 16.

**Table 16. Sieving tower analysis.**

| Sample ID | Powder fraction passing the sieve, wt % |
|---|---|
| C2 (comparative) | 89.2 |
| PAM61 | 94.5 |
| C3 (comparative) | 83.4 |
| PAM62 | 94.4 |
| C4 (comparative) | 89.9 |
| PAM63 | 95.3 |

Compared to the corresponding comparative sample C2 without divalent cations and without inorganic phosphate, inventive pouch product PAM61 was found to have significantly improved processability as evident by the sieving test results. Similarly, inventive pouch product PAM62 was found to have significantly improved processability compared to corresponding comparative sample C3, and inventive pouch product PAM63 was found to have significantly improved processability compared to corresponding comparative sample C4.

## Claims

1. An oral nicotine pouch composition comprising
nicotine,
water in an amount of at least 5% by weight of the pouch composition, alginate,
inorganic divalent cations, and
at least one pH regulating agent comprising inorganic phosphate.

2. The oral nicotine pouch composition according to claim 1, wherein the phosphate comprises at least two different phosphates.

3. The oral nicotine pouch composition according to claim 1 or 2, wherein the phosphate comprises a combination of monophosphate and polyphosphate, such as pyrophosphate.

4. The oral nicotine pouch composition according to any of claims 1-3, wherein the phosphate comprises phosphate provided as a salt with an alkali metal, such as sodium or potassium.

5. The oral nicotine pouch composition according to any of claims 1-4, wherein the phosphate is provided as a salt in an amount of at least 0.1% by weight of the pouch composition, such as at least 0.2% by weight of the pouch composition, such as at least 0.5% by weight of the pouch composition, such as at least 1.0% by weight of the pouch composition.

6. The oral nicotine pouch composition according to any of claims 1-5, wherein the pouch composition comprises alginate in an amount of at least 0.1% by weight of the pouch composition, such as at least 0.2% by weight of the pouch composition, such as at least 0.3% by weight of the pouch composition, such as at least 0.4% by weight of the pouch composition.

7. The oral nicotine pouch composition according to any of claims 1-6, wherein the alginate is provided as a salt with an ion selected from the group consisting of alkali metal alginate, such as sodium alginate or potassium alginate, or any combinations thereof.

8. The oral nicotine pouch composition according to any of claims 1-7, wherein the inorganic divalent cations are selected from the group consisting of divalent cations of calcium, magnesium, iron, zinc, and any combination thereof.

9. The oral nicotine pouch composition according to any of claims 1-8, wherein the inorganic divalent cations are provided as an inorganic salt.

10. The oral nicotine pouch composition according to any of claims 1-9, wherein the inorganic divalent cations are provided as an inorganic salt comprising inorganic anions selected from the group consisting of chloride, bromide, nitrate, sulfate, hydrogen carbonate, hydrogen phosphate, oxide, hydroxide, and any combination thereof.

11. The oral nicotine pouch composition according to any of claims 1-10, wherein the inorganic divalent cations are provided as a salt in an amount of at least 0.1% by of the pouch composition, such as at least 0.2% by weight of the pouch composition, such as at least 0.3% by weight of the pouch composition, such as at least 0.4% by weight of the pouch composition.

12. The oral nicotine pouch composition according to any of claims 1-11, wherein the pouch composition comprises said alginate in a weight ratio of 1:2 to 3:1 relative to the amount of divalent cation, such as 1:1 to 2:1 relative to the amount of divalent cation.

13. The oral nicotine pouch composition according to any of claims 1-12, wherein the pouch composition comprises said alginate in a weight ratio of 1:5 to 3:1 relative to the amount of phosphate, such as 1:3 to 1: 1 relative to the amount of phosphate, such as 1:2 to 2:3 relative to the amount of phosphate.

14. The oral nicotine pouch composition according to any of claims 1-13, wherein the at least one pH regulating agent comprises at least one further pH regulating agent.

15. The oral nicotine pouch composition according to any of claims 1-14, wherein the at least one further pH regulating agent is selected from the group consisting of sodium carbonate, sodium bicarbonate, potassium carbonate, and magnesium carbonate, potassium bicarbonate, trometamol, or any combination thereof.

16. The oral nicotine pouch composition according to any of claims 1-15, wherein the nicotine is selected from the group consisting of a nicotine salt, nicotine free base, a nicotine-ion exchange resin combination, a nicotine inclusion complex or nicotine in any non-covalent binding; nicotine bound to zeolites, nicotine bound to cellulose, such as microcrystalline cellulose, starch microspheres, and mixtures thereof.

17. The oral nicotine pouch composition according to any of claims 1-16, wherein the nicotine comprises nicotine bound to an ion exchange resin.

18. The oral nicotine pouch composition according to any of claims 1-17, wherein the pouch composition comprises at least one sugar alcohol.

19. The oral nicotine pouch composition according to any of claims 1-18, wherein the pouch composition comprises water-insoluble fiber.

20. The oral nicotine pouch composition according to any of claims 1-19, wherein the pouch composition is free of tobacco fibers.

21. A nicotine pouched product comprising a pouch composition according to any of claims 1-20 and a saliva-permeable pouch enclosing said pouch composition.

## Patentansprüche

1. Orale Nikotinbeutelzusammensetzung umfassend
Nikotin,
Wasser in einer Menge von mindestens 5 Gew.-% der Beutelzusammensetzung,
Alginat,
anorganische zweiwertige Kationen, und
mindestens ein pH-regulierendes Mittel, umfassend anorganisches Phosphat.

2. Orale Nikotinbeutelzusammensetzung nach Anspruch 1, wobei das Phosphat mindestens zwei verschiedene Phosphate umfasst.

3. Orale Nikotinbeutelzusammensetzung nach Anspruch 1 oder 2, wobei das Phosphat eine Kombination aus Monophosphat und Polyphosphat, wie Pyrophosphat, umfasst.

4. Orale Nikotinbeutelzusammensetzung nach einem der Ansprüche 1 bis 3, wobei das Phosphat Phosphat umfasst, das als ein Salz mit einem Alkalimetall bereitgestellt ist, wie Natrium oder Kalium.

5. Orale Nikotinbeutelzusammensetzung nach einem der Ansprüche 1 bis 4, wobei das Phosphat als ein Salz in einer Menge von mindestens 0,1 Gew.-% der Beutelzusammensetzung, wie mindestens 0,2 Gew.-% der Beutelzusammensetzung, wie mindestens 0,5 Gew.-% der Beutelzusammensetzung, wie mindestens 1,0 Gew.-% der Beutelzusammensetzung bereitgestellt ist.

6. Orale Nikotinbeutelzusammensetzung nach einem der Ansprüche 1 bis 5, wobei die Beutelzusammensetzung Alginat in einer Menge von mindestens 0,1 Gew.-% der Beutelzusammensetzung, wie mindestens 0,2 Gew.-% der Beutelzusammensetzung, wie mindestens 0,3 Gew.-% der Beutelzusammensetzung, wie etwa 0,4 Gew.-% der Beutelzusammensetzung umfasst.

7. Orale Nikotinbeutelzusammensetzung nach einem der Ansprüche 1 bis 6, wobei das Alginat als ein Salz mit einem Ion bereitgestellt ist, das aus der Gruppe ausgewählt ist, bestehend aus Alkalimetallalginat, wie Natriumalginat oder Kaliumalginat, oder beliebigen Kombinationen davon.

8. Orale Nikotinbeutelzusammensetzung nach einem der Ansprüche 1 bis 7, wobei die anorganischen zweiwertigen Kationen aus der Gruppe ausgewählt sind, bestehend aus zweiwertigen Kationen von Calcium, Magnesium, Eisen, Zink und einer beliebigen Kombination davon.

9. Orale Nikotinbeutelzusammensetzung nach einem der Ansprüche 1 bis 8, wobei die anorganischen zweiwertigen Kationen als ein anorganisches Salz bereitgestellt sind.

10. Orale Nikotinbeutelzusammensetzung nach einem der Ansprüche 1 bis 9, wobei die anorganischen zweiwertigen Kationen als ein anorganisches Salz bereitgestellt sind, umfassend anorganische Anionen, die aus der Gruppe ausgewählt sind, bestehend aus Chlorid, Bromid, Nitrat, Sulfat, Hydrogencarbonat, Hydrogenphosphat, Oxid, Hydroxid und einer beliebigen Kombination davon.

11. Orale Nikotinbeutelzusammensetzung nach einem der Ansprüche 1 bis 10, wobei die anorganischen zweiwertigen Kationen als ein Salz in einer Menge von mindestens 0,1 Gew.-% der Beutelzusammensetzung, wie mindestens 0,2 Gew.-% der Beutelzusammensetzung, wie mindestens 0,3 Gew.-% der Beutelzusammensetzung, wie etwa 0,4 Gew.-% der Beutelzusammensetzung bereitgestellt sind.

12. Orale Nikotinbeutelzusammensetzung nach einem der Ansprüche 1 bis 11, wobei die Beutelzusammensetzung das Alginat in einem Gewichtsverhältnis von 1 : 2 bis 3 : 1 relativ zu der Menge an zweiwertigem Kation, wie 1 : 1 bis 2 : 1 relativ zu der Menge an zweiwertigem Kation umfasst.

13. Orale Nikotinbeutelzusammensetzung nach einem der Ansprüche 1 bis 12, wobei die Beutelzusammensetzung das Alginat in einem Gewichtsverhältnis von 1 : 5 bis 3 : 1 relativ zu der Menge an Phosphat, wie 1 : 3 bis 1 : 1 relativ zu der Menge an Phosphat, wie 1 : 2 bis 2 : 3 relativ zu der Menge an Phosphat umfasst.

14. Orale Nikotinbeutelzusammensetzung nach einem der Ansprüche 1 bis 13, wobei das mindestens eine pH-regulierende Mittel mindestens ein weiteres pH-regulierendes Mittel umfasst.

15. Orale Nikotinbeutelzusammensetzung nach einem der Ansprüche 1 bis 14, wobei das mindestens eine weitere pH-regulierende Mittel aus der Gruppe ausgewählt ist, bestehend aus Natriumcarbonat, Natriumbicarbonat, Kaliumcarbonat und Magnesiumcarbonat, Kaliumbicarbonat, Trometamol oder einer beliebigen Kombination davon.

16. Orale Nikotinbeutelzusammensetzung nach einem der Ansprüche 1 bis 15, wobei das Nikotin aus der Gruppe ausgewählt ist, bestehend aus einem Nikotinsalz, nikotinfreier Base, einer Nikotin-Ionenaustauscherharz-Kombination, einem Nikotin-Einschlusskomplex oder Nikotin in einer nicht-kovalenten Bindung; Nikotin, das an Zeolithe gebunden ist, Nikotin, das an Cellulose gebunden ist, wie mikrokristalline Cellulose, Stärkemikrosphären, und Mischungen davon.

17. Orale Nikotinbeutelzusammensetzung nach einem der Ansprüche 1 bis 16, wobei das Nikotin Nikotin umfasst, das an ein Ionenaustauscherharz gebunden ist.

18. Orale Nikotinbeutelzusammensetzung nach einem der Ansprüche 1 bis 17, wobei die Beutelzusammensetzung mindestens einen Zuckeralkohol umfasst.

19. Orale Nikotinbeutelzusammensetzung nach einem der Ansprüche 1 bis 18, wobei die Beutelzusammensetzung wasserunlösliche Fasern umfasst.

20. Orale Nikotinbeutelzusammensetzung nach einem der Ansprüche 1 bis 19, wobei die Beutelzusammensetzung frei von Tabakfasern ist.

21. Nikotinbeutelprodukt, umfassend eine Beutelzusammensetzung nach einem der Ansprüche 1 bis 20 und einen speicheldurchlässigen Beutel, der die Beutelzusammensetzung umschließt.

## Revendications

1. Composition orale de sachet de nicotine comprenant
de la nicotine,
de l'eau en une quantité d'au moins 5 % en poids de la composition de sachet,
de l'alginate,
des cations divalents inorganiques, et
au moins un agent de régulation du pH comprenant du phosphate inorganique.

2. Composition orale de sachet de nicotine selon la revendication 1, dans laquelle le phosphate comprend au moins deux phosphates différents.

3. Composition orale de sachet de nicotine selon la revendication 1 ou 2, dans laquelle le phosphate comprend une combinaison de monophosphate et de polyphosphate, telle que du pyrophosphate.

4. Composition orale de sachet de nicotine selon l'une quelconque des revendications 1 à 3, dans laquelle le phosphate comprend du phosphate fourni sous forme de sel avec un métal alcalin, tel que du sodium ou potassium.

5. Composition orale de sachet de nicotine selon l'une quelconque des revendications 1 à 4, dans laquelle le phosphate est fourni sous forme de sel en une quantité d'au moins 0,1 % en poids de la composition de sachet, telle qu'au moins 0,2 % en poids de la composition de sachet, telle qu'au moins 0,5 % en poids de la composition de sachet, telle qu'au moins 1,0 % en poids de la composition de sachet.

6. Composition orale de sachet de nicotine selon l'une quelconque des revendications 1 à 5, dans laquelle la composition de sachet comprend de l'alginate en une quantité d'au moins 0,1 % en poids de la composition de sachet, telle qu'au moins 0,2 % en poids de la composition de sachet, telle qu'au moins 0,3 % en poids de la composition de sachet, telle qu'au moins 0,4 % en poids de la composition de sachet.

7. Composition orale de sachet de nicotine selon l'une quelconque des revendications 1 à 6, dans laquelle l'alginate est fourni sous forme de sel avec un ion choisi dans le groupe constitué d'alginate de métal alcalin, tel que l'alginate de sodium ou l'alginate de potassium, ou leurs combinaisons quelconques.

8. Composition orale de sachet de nicotine selon l'une quelconque des revendications 1 à 7, dans laquelle les cations divalents inorganiques sont choisis dans le groupe constitué de cations divalents de calcium, de magnésium, de fer, de zinc et leur combinaison quelconque.

9. Composition orale de sachet de nicotine selon l'une quelconque des revendications 1 à 8, dans laquelle les cations divalents inorganiques sont fournis sous forme de sel inorganique.

10. Composition orale de sachet de nicotine selon l'une quelconque des revendications 1 à 9, dans laquelle les cations divalents inorganiques sont fournis sous forme de sel inorganique comprenant des anions inorganiques choisis dans le groupe constitué de chlorure, bromure, nitrate, sulfate, hydrogène carbonate, hydrogène phosphate, oxyde, hydroxyde et leur combinaison quelconque.

11. Composition orale de sachet de nicotine selon l'une quelconque des revendications 1 à 10, dans laquelle les cations divalents inorganiques sont fournis sous forme de sel en une quantité d'au moins 0,1 % en poids de la composition de sachet, telle qu'au moins 0,2 % en poids de la composition de sachet, telle qu'au moins 0,3 % en poids de la composition de sachet, telle qu'au moins 0,4 % en poids de la composition de sachet.

12. Composition orale de sachet de nicotine selon l'une quelconque des revendications 1 à 11, dans laquelle la composition de sachet comprend ledit alginate dans un rapport en poids de 1:2 à 3:1 par rapport à la quantité de cation divalent, tel que de 1:1 à 2:1 par rapport à la quantité de cation divalent.

13. Composition orale de sachet de nicotine selon l'une quelconque des revendications 1 à 12, dans laquelle la composition de sachet comprend ledit alginate dans un rapport en poids de 1:5 à 3:1 par rapport à la quantité de phosphate, tel que 1:3 à 1:1 par rapport à la quantité de phosphate, tel que 1:2 à 2:3 par rapport à la quantité de phosphate.

14. Composition orale de sachet de nicotine selon l'une quelconque des revendications 1 à 13, dans laquelle l'au moins un agent de régulation du pH comprend au moins un agent de régulation du pH supplémentaire.

15. Composition orale de sachet de nicotine selon l'une quelconque des revendications 1 à 14, dans laquelle l'au moins un agent de régulation de pH supplémentaire est choisi dans le groupe constitué de carbonate de sodium, bicarbonate de sodium, carbonate de potassium et carbonate de magnésium, bicarbonate de potassium, trométamol ou leur combinaison quelconque.

16. Composition orale de sachet de nicotine selon l'une quelconque des revendications 1 à 15, dans laquelle la nicotine est choisie dans le groupe constitué d'un sel de nicotine, d'une base libre de nicotine, d'une combinaison de résine échangeuse d'ions et de nicotine, d'un complexe d'inclusion de nicotine ou nicotine selon une liaison non covalente quelconque ; nicotine liée à des zéolites, nicotine liée à de la cellulose, telle que de la cellulose microcristalline, ou des microsphères d'amidon et leurs mélanges.

17. Composition orale de sachet de nicotine selon l'une quelconque des revendications 1 à 16, dans laquelle la nicotine comprend de la nicotine liée à une résine échangeuse d'ions.

18. Composition orale de sachet de nicotine selon l'une quelconque des revendications 1 à 17, dans laquelle la composition de sachet comprend au moins un alcool de sucre.

19. Composition orale de sachet de nicotine selon l'une quelconque des revendications 1 à 18, dans laquelle la composition de sachet comprend des fibres hydro-insoluble.

20. Composition orale de sachet de nicotine selon l'une quelconque des revendications 1 à 19, dans laquelle la composition de sachet est exempte de fibres de tabac.

21. Produit en sachet de nicotine comprenant une composition de sachet selon l'une quelconque des revendications 1 à 20 et un sachet perméable à la salive renfermant ladite composition de sachet.
